# EUROPEAN PATENT APPLICATION

(11) **EP 3 106 144 A1**
(43) Date of publication of application: **21.12.2016**
(21) Application number: 16174911.4
(22) Date of filing: 17.06.2016
(51) Int. Cl.: A61H 39/02, A61B 5/053, A61B 5/12

(54) **APPARATUS FOR THE TREATMENT OF PSYCHO-PHYSICAL AILMENTS**

(30) Priority: 17.06.2015 IT UB20151439
(71) Applicant: Dynamica Acustica S.r.l., 20122 Milano (IT)
(72) Inventor: PISANO, Gianpaolo, I - 20090 BUCCINASCO (Milano) (IT)
(74) Representative: Lunati & Mazzoni S.r.L.

(57) **Abstract**

An apparatus for the treatment of psycho-physical ailments (1) is provided, comprising: stress means (2) of the human body (U), suitable to apply to the human body (U) predetermined stresses, and first measurement means (3), suitable to measure the condition of the stress means (2), second measurement means (4) of the reactance of the human body (U), control means (5), functionally connected to said first and second measurement means (3 and 4), and suitable to correlate values of said second measurement means (4) to values measured at the same moment by said first measurement means (3).

## Description

The present invention relates to an apparatus for the treatment of psychophysical ailments of the type as recited in the preamble of the first claim.

Various types of oriental medicine are currently known of, in recent decades also recognised in the West.

For example various medicines are known of based on the use of so-called meridians of the human body, which are energy channels running along the human body itself.

In particular, the known practice of acupuncture, recognised by the WHO and used for centuries in the East, is based on said meridians. Along said meridians very thin needles are in fact inserted in order to promote the health and welfare of the individual: according to traditional Chinese medicine, stimulating these points can correct imbalances in the flow of energy through the meridians.

The meridians may also be stimulated by other elements that create point stresses, such as laser or point heating elements.

Other types of oriental medicine provide for listening to particular sounds, in particular comprised in a reduced range of frequencies.

Other types of Oriental medicine provide for the frequent practice of oriental style meditation, which aims to stop the mind at a static image and not allow it to act without control.

Said practices lead to a state of well-being or balance, which make it possible to cure or obtain a major improvement for many diseases and for many psycho-physical ailments.

Moreover, said traditional oriental therapies have substantially no side effects.

The prior art described has several significant drawbacks.

In particular, said therapies do not always give optimal results.

For example, some speculate that the meridians may vary from one individual to another and change in sick people.

In addition, many treatments are difficult to apply to Western patients, who are used to getting an immediate experimental response. The lack of such immediate response can cause disbelief and discontent and lead to an early abandonment of therapy, or practice of the same without commitment.

Said lack of an immediate objective response also leads to a reduced diffusion of traditional oriental medicine in the West.

In this situation the technical purpose of the present invention is to devise an apparatus for the treatment of psycho-physical ailments able to substantially overcome the drawbacks mentioned above.

In the context of this technical task one important aim of the invention is to obtain an apparatus for the treatment of psycho-physical ailments which is able to achieve more constant results.

Another important object of the invention is to make an apparatus for the treatment of psycho-physical ailments able to offer a better immediate measurement of the effects on the human body.

The technical purpose and specified aims are achieved by an apparatus for the treatment of psycho-physical ailments as claimed in the appended Claim 1. Preferred embodiment examples are described in the dependent claims.

The characteristics and advantages of the invention are clearly evident from the following detailed description of preferred embodiments thereof, with reference to the accompanying drawings, in which:
**Fig. 1** shows a first example of apparatus for the treatment of psycho-physical ailments and
**Fig. 2** shows a second example of apparatus for the treatment of psycho-physical ailments.

With reference to the Drawings, reference numeral **1** globally denotes the apparatus for the treatment of psycho-physical ailments according to the invention. It is suitable to treat a human being or human body **U.**

The apparatus 1 comprises stress means **2** of the human body U, suitable to apply predetermined stresses to the human body U, and first measurement means **3,** suitable to measure the condition of the stress means 2.

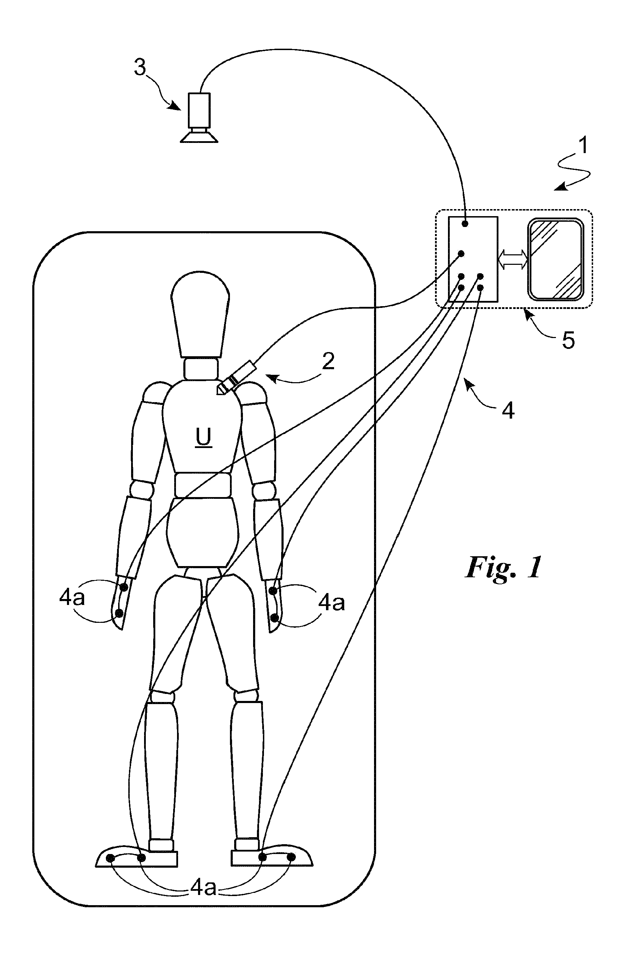

In a first example, shown in Fig. 1, the stress means 2 are suitable to apply a substantially point-shaped stimulus. In this example the first measurement means 3 are preferably suitable to measure the position on the human body U of the stress means 2.

More in detail the stress means 2 may be suitable to emit a laser beam. In particular, such laser beam is obtained by means of a He-Ne laser, suitably with powers from 5 to 7 mV.

Alternatively, again with reference to the example of Fig. 1, the stress means 2 are suitable to emit heat through a substantially point-shaped source, similar for example to the tip of a pen or the like. Such heat may be simply obtained by means of an electrical resistance by the Joule or other effect.

In another alternative, the stress means 2 consist of injections of distilled water. The stress means 2 are thus preferably stick-shaped.

In the example of figure 1 the first measurement means 3 measure the position on the human body of the stress means 2 by means of solutions known per se. For example by means of a camera that detects the position of the human body U and the measuring means. The said camera may be aided by optical indicators, such as particular colours of the stick-type stress means 2 or other indicators of a particular colour applied on the human body, or by a suitably coloured bed contrasting with the human body. Alternatively, the measurement means measure the position 3 on the human body U of the stress means 2 by means of accelerometers, magnetic elements or by simple paths drawn on the human body U and filmed by cameras or video cameras and digitalised if necessary by appropriate software.

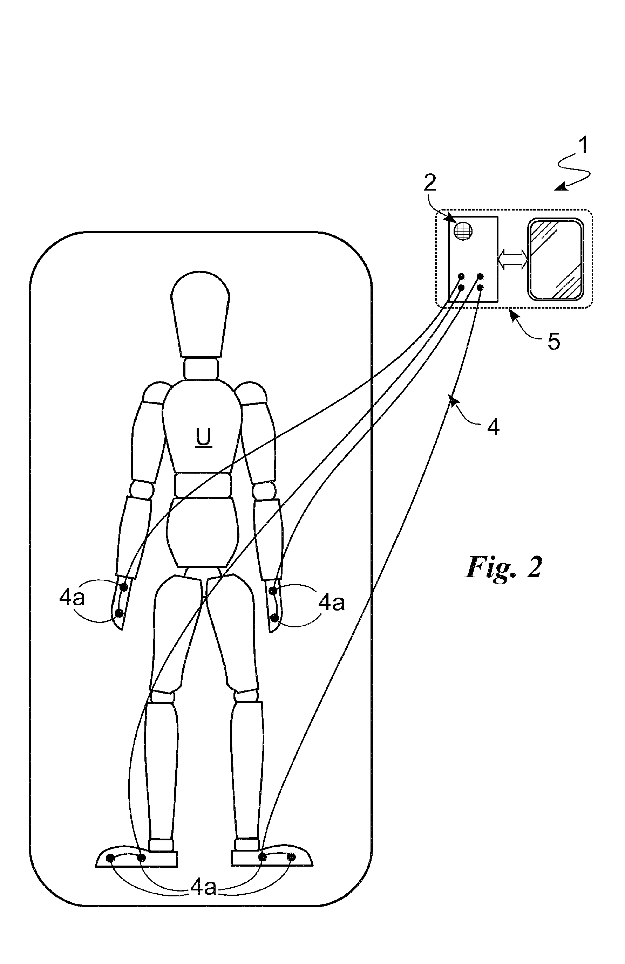

In a second example, shown in Fig. 2, the stress means 2 are suitable to emit sounds, in particular sounds in a low frequency range and in particular substantially mono-frequency sounds of predetermined frequencies, such as the sounds produced by a tuning fork or the like. They therefore substantially consist of loudspeakers.

In this case the first measurement means 3 are suitable to measure or more simply record or catalogue or store the sound emitted by the stress means 2 in the specific instant.

The apparatus 1 may further comprise both the stress means 2 and measurement means 3 of the two examples of Fig. 1 and Fig. 2.

The apparatus for the treatment of psycho-physical ailments 1 further comprises second measurement means **4** suitable for measuring at least indirectly, the reactance of said human body U.

The said second measurement means 4 preferably consist of a bioelectrical impedance meter known per se. In particular, the bioelectrical impedance meter measures the reactance of the body to the passage of an electrical current at low power and high frequency (10-100 kHz, preferably 50 kHz). For example it is the bioelectrical impedance meter BIA 101 manufactured by Akern Srl. It preferably comprises two electrodes **4a,** one positive and one negative, per limb of the human body U.

The apparatus for the treatment of psycho-physical ailments 1 comprises then control means **5,** functionally connected to the first and second measurement means 3 and 4, and suitable to correlate values of said second measurement means 4 to values measured at the same moment by said first measurement means 3.

The control means 5, which may consist of a computer, tablet, or similar device, or two mutually connected devices, are then preferably electrically connected via electrical cables to the electrodes 4a or to the remaining part of the second measurement means 4. The control means 5 are also connected to the loudspeakers, or to the laser beam or heat emitters. The loudspeakers are preferably integrated in the computer or the like, while the laser beam or heat emittors are connected via electric cable or in wireless manner with known connections.

The control means 5 then correlate the reactance measured by the second measurement means 4 with the measurement performed by the first measurement means 3, and thus the sound or the position of the laser or heat emitters. At each specific sound to be played or at each position of the laser or heat emitter the corresponding reactance is thus accurately identified.

The measuring means 4 may be, alternatively, suitable to measure only the impedance of the human body, or bioimpedance. In this case the reactance is in any case measured indirectly, in fact, given that the resistance remains fixed, a minimum in impedance corresponds to a minimum in reactance.

In particular, the control means 5 are suitable to correlate minimum reactance values of the second measurement means 4 to values measured in the same instants by said first measurement means 3.

These minimum reactance values are preferably lower by a delta greater than 5 Ω, and more preferably of 20 Ω, compared to the reactance values measured in the rest of the human body. The said delta can also be total, in the sense that the reactance may reach substantially zero values.

The control means 5 are thus suitable to record positions on the human body U under examination, and then to map the meridians of said human body U under examination.

The functioning of the apparatus for the treatment of psycho-physical ailments 1, described above in structural terms, is as follows. Said functioning also defines a new procedure.

The patient to be treated is laid down in the vicinity of the apparatus 1, or vice versa, the apparatus 1 is placed in the vicinity of a patient already lying down or seated.

The stress means 2 are then activated and an attendant places the laser or heat source at or in proximity of the meridians, or of the points of the human body U where said meridians should be present. Alternatively the stress means 2 propose sounds to the patient.

Said sounds or the positions of the stress means 2 are in the meantime measured by the first measurement means 3 and stored or controlled by the control means 5. Meanwhile, the second control means 4 measure the reactance of the human body, constantly controlled and monitored by the control means 5.

The applicant has surprisingly discovered that in some cases, with suitable stress means 2, and preferably when the human body U is sufficiently and correctly hydrated the reactance of the human body U drops considerably and in particular to below the previously specified delta values, i.e. greater than 5 Ω, and even higher than 20 Ω. In many cases the reactance reaches a substantially zero value. In such cases the control means 5 select and immediately save the value measured by the first measurement means 3, then the position of the laser or heat or the sound emitted.

The applicant has also surprisingly found that such points in which the reactance falls correspond to important points for psycho-physical health, or simply for the well-being or pleasure of the individual treated.

In particular said low reactance points correspond to the correct and custom mapping of meridians on the individual treated.

Furthermore, the sounds emitted and recorded or stored in corresponding low reactance points correspond to sounds which, if listened to for long or even short periods of time, preferably periodically, can rebalance the patient's physcho-physical health. In particular such a method, performed with the apparatus of Fig. 2, has been confidentially successfully tested on patients with problems such as in particular tinnitus, dysrhythmia or jet lag problems and other disorders related to the ear.

The apparatus for the treatment of psycho-physical ailments 1 according to the invention achieves important advantages.

In fact, the apparatus for the treatment of psychophysical ailments is able to achieve a constancy of results and to give a better measure of the immediate effects on the human body, directly measurable and practically visible through the lowering of the reactance of the human body.

Furthermore, the apparatus 1 makes it possible to correctly remap the meridians of the human body.

Lastly, said apparatus makes it possible to cure the disorder of tinnitus, which is not considered a disease, or other disorders related to hearing.

Variations may be made to the invention without departing from the scope of the inventive concept defined in the claims.

In said sphere all the details may be replaced with equivalent elements and the materials, shapes and dimensions may be as desired.

## Claims

1. Apparatus for the treatment of psycho-physical ailments (1) comprising:
- stress means (2) of the human body (U), suitable to apply to said human body (U) predetermined stress,
- first measurement means (3), designed to measure the condition of such stress means (2),
- second measurement means (4) of the reactance of said human body (U),
- control means (5), functionally connected to said first and second measurement means (3 and 4), and suitable to correlate values of said second measurement means (4) to values measured at the same moment by said first measurement means (3).

2. Apparatus (1) according to the previous claim, wherein said control means (5) are suitable to correlate values of said second measurement means (4) one delta below and 5 Ω above the values measured in the same moment by said first measurement means (3).

3. Apparatus (1) according to one or more of the previous claims, in which the stress means (2) are suitable to apply a substantially point-shaped stimulus.

4. Apparatus (1) according to the previous claim, wherein said stress means (2) are suitable to emit a laser beam.

5. Apparatus (1) according to the previous claim, wherein said stress means (2) are suitable to emit heat.

6. Apparatus (1) according to one or more of the previous claims, wherein said first measurement means (3) are suitable to measure the position on the human body of said stress means (2).

7. Apparatus (1) according to the previous claim, wherein said control means (5) are designed to map the meridians of said human body (U) being examined.

8. Apparatus (1) according to one or more of the previous claims, wherein said stress means (2) are suitable to emit sounds.

9. Apparatus (1) according to the previous claim, wherein said sounds are single-frequency sounds of predetermined frequencies.

10. Method of treatment of tinnitus comprising: listening to predetermined sounds, the simultaneous measurement of the reactance of the human body, the selection of sounds which led to a lowering of said reactance of the human body, periodic listening to said sounds.
